# EUROPEAN PATENT SPECIFICATION

(11) **EP 2 772 183 B1**
(45) Date of publication and mention of the grant of the patent: **15.08.2018**
(21) Application number: 14155450.1
(22) Date of filing: 17.02.2014
(51) Int. Cl.: A61B 1/00

(54) **Electronic endoscope and method of manufacturing electronic endoscope**
Elektronisches Endoskop und Verfahren zur Herstellung eines elektronischen Endoskops
Endoscope électronique et procédé de fabrication d'endoscope électronique

(30) Priority: 27.02.2013 JP 2013036750
(43) Date of publication of application: 03.09.2014
(73) Proprietor: FUJIFILM Corporation, Minato-ku Tokyo 106-8620 (JP)
(72) Inventor: Endo, Keisuke, Kanagawa 250-0193 (JP)
(74) Representative: Klunker IP Patentanwälte PartG mbB

(56) References cited:
- EP-A1- 0 461 002
- WO-A2-2007/092533
- JP-A- 2010 146 739
- US-A- 4 151 364
- US-A1- 2010 261 961

## Description

### BACKGROUND OF THE INVENTION

### Field of the Invention

The present invention relates to an electronic endoscope and a method of manufacturing an electronic endoscope and, more particularly, to a connection technique for terminal pins of an electronic endoscope having an airtight structure.

### Description of the Related Art

Medical endoscopes are each used to observe organs by inserting an insertion part thereof into a body cavity and to perform various medical procedures or treatments using a treatment tool inserted into a treatment tool insertion channel thereof. Hence, in the case where an endoscope used once is reused for another patient, the endoscope needs to be disinfected and sterilized after the end of an examination/treatment, in order to prevent an infection between patients through the endoscope.

In the case of disinfection and sterilization using a washing liquid, there are disadvantages that the disinfection work is complicated and that the waste liquid disposal of the washing liquid costs an enormous amount of money.

Recently, high-pressure high-temperature steam sterilization (autoclave) that does not require complicated work has been becoming the mainstream of a technique of sterilizing medical tools. In the case where an electronic endoscope is sterilized using the autoclave, the following problem arises when even a little amount of moisture (water vapor) of a damp atmosphere or the like infiltrates a portion around an electrical circuit of a scope distal end part. The moisture fogs an optical objective lens from the inside, and corrodes, for example, a circuit board on which an image pickup element and a drive circuit component that processes signals from the image pickup element are mounted, whereby the moisture causes a decrease in quality of taken images and a breakdown due to short-circuiting or the like. In a structure proposed to deal with this problem, the optical objective lens, the image pickup element, and the circuit board are sealed by an airtight container and an airtight terminal, in order to prevent moisture infiltration and thus prevent a decrease in function and a deterioration in component (Japanese Patent Application Laid-Open No. 10-234649).

Meanwhile, along with a reduction in electronic endoscope diameter, such an airtight structure is required to be downsized. In order to draw signal lines from the image pickup element to the outside of the airtight structure, it is necessary to respectively connect terminal pins of the airtight terminal and the signal lines.

With regard to electrical connection between the two members, a large number of methods of connecting electric wires to each other are proposed. Japanese Patent Application Laid-Open No. 2006-185902 describes a sleeve for electric wires with insulating coating, as a connection tool (sleeve) for directly connecting the electric wires to each other. The inside of this sleeve is filled with an electrically conductive compound, and an annular projection is formed on an inner wall surface using a waterproof rubber member such that this compound does not leak to the outside at the time of crimping the electric wires.

Japanese Patent Application Laid-Open No. 2009-176729 describes that a sleeve body made of aluminum is squashed through compression with the intermediation of insulating coating, whereby electric wires are connected to each other. The sleeve body is held and compressed with a die by a length three times or more of the thickness of the coating, and hence a breakdown of the insulating coating is suppressed from occurring.

Japanese Patent Application Laid-Open No. 2010-015900 describes a method of connecting electric wires to each other. In this method, when two or more electric wires are fixed by a crimp terminal, conductors of the electric wires are held by a first conductor holding part and a second conductor holding part that are different in height.

Japanese Patent Application Laid-Open No. 2010-146739 describes that a sheet-like metal material is cylindrically rolled on the inner surface of a sleeve so that a diameter of a central portion of the sleeve is made smaller than both ends, whereby the reliability of connection between two electric wires inserted in the sleeve is enhanced.

US 2010/261961 A1 discloses an electronic endoscope and a method of manufacturing an electronic endoscope according to the preamble of claim 1 and claim 12, repectively. According to figure 5D of this document, the signal lines of the signal line cable are received by a connector body and are electrically connected to cup-shaped female pins into which the terminal pins are inserted for connecting the signal lines with the terminal pins.

### SUMMARY OF THE INVENTION

Meanwhile, in the case of electronic endoscopes, 15 to 20 signal lines are necessary for signal processing. If an airtight structure is downsized, electrode terminals having extremely small diameters are closely packed. Work of individually connecting each terminal pin and each signal line is difficult, and this causes problems such as a decrease in yield due to a breakdown during connection and an increase in cost.

In the connection method described in Japanese Patent Application Laid-Open No. 2006-185902, the electric wires inserted into the sleeve from both ends are swaged by crimping. Hence, there is a problem that it is difficult to respectively connect the closely packed electrode terminals having extremely small diameters and the electric wires.

In the connection methods described in Japanese Patent Application Laid-Open Nos. 2009-176729 and 2010-015900, the electric wires are connected to each other by crimping. Hence, as long as a crimping step and such other steps are necessary, there is a problem that it is difficult to respectively connect the closely packed electrode terminals having extremely small diameters and the electric wires.

In the connection method described in Japanese Patent Application Laid-Open No. 2010-146739, the sheet-like metal material is rolled into a cylinder so that the diameter of the central portion of the sleeve is made smaller. Hence, there is a problem that it is difficult to apply this method to the closely packed electrode terminals having extremely small diameters.

The present invention, which has been made in view of the above-mentioned circumstances, aims to provide an electronic endoscope having an airtight structure that enables connection between closely packed terminal pins having extremely small diameters and signal lines, as well as a method of manufacturing the electronic endoscope.

An electronic endoscope according to a first aspect of the present invention includes: a plurality of internal signal lines which are electrically connected to an image pickup element that receives incident light, a circuit board on which the image pickup element and a drive circuit component for the image pickup element are mounted; an airtight structure including an airtight container and an airtight terminal through which a plurality of terminal pins penetrate, the airtight structure in which the image pickup element, the circuit board and the plurality of the internal signal lines are sealed; a signal line cable including a plurality of signal lines, the signal line cable being placed outside of the airtight structure; and a plurality of pipe-shaped electrically conductive members which electrically connect the plurality of signal lines of the signal line cable and the plurality of terminal pins placed outside of the airtight structure, respectively.

Preferably, the electronic endoscope further includes an electrically conductive adhesive which is used for at least one of a connection between the signal line and the electrically conductive member, and a connection between the terminal pin located outside of the airtight structure and the electrically conductive member.

Preferably, the electronic endoscope further includes an insulating tube that coats the signal line, the terminal pin located outside of the airtight structure, and the electrically conductive member.

Preferably, the electronic endoscope further includes a plurality of pipe-shaped electrically conductive members which electrically connect the plurality of internal signal lines and the plurality of terminal pins located inside of the airtight structure, respectively.

Preferably, the electronic endoscope further includes an electrically conductive adhesive which is used for at least one of a connection between the internal signal line and the electrically conductive member, and a connection between the terminal pin located inside of the airtight structure and the electrically conductive member.

Preferably, the electronic endoscope further includes a heat-shrinkable tube that coats the internal signal line, the terminal pin located inside of the airtight structure, and the electrically conductive member.

Preferably, the electrically conductive adhesive has an upper temperature limit of 130°C or higher.

Preferably, the electrically conductive member includes a splitting slit (64) on a side to which the terminal pin is connected.

Preferably, the electrically conductive member has an inner diameter that is smaller in a center than at both ends.

Preferably, the electrically conductive member has a tapered shape having an inner diameter that gradually becomes smaller from a side to which the terminal pin is connected toward a side opposite thereto.

Preferably, the electrically conductive member has a surface that has been subjected to an insulation process.

A method of manufacturing an electronic endoscope according to a second aspect of the present invention includes at least: preparing an image pickup element that receives incident light, a circuit board on which the image pickup element and a drive circuit component for the image pickup element are mounted, a plurality of internal signal lines, an airtight terminal through which a plurality of terminal pins penetrate, an airtight container, and a signal line cable including a plurality of signal lines; airtightly sealing the image pickup element, the circuit board, and the plurality of internal signal lines by means of the airtight terminal and the airtight container; and electrically connecting each of the plurality of signal lines and each of the plurality of terminal pins by means of a pipe-shaped electrically conductive member.

The electrically connecting each of the plurality of signal lines and each of the plurality of terminal pins by means of the pipe-shaped electrically conductive member may be performed before the hermetic sealing, or may be performed after the hermetic sealing.

Preferably, the method of manufacturing an electronic endoscope further includes: electrically connecting the plurality of internal signal lines and the circuit board; and electrically connecting each of the plurality of internal signal lines and each of the plurality of terminal pins by means of a pipe-shaped electrically conductive member.

Preferably, the method of manufacturing an electronic endoscope further includes filling insides of both end portions of the electrically conductive member with an electrically conductive adhesive, and after the filling with the electrically conductive adhesive, performing at least one of: electrically connecting each of the plurality of internal signal lines and each of the plurality of terminal pins by means of the pipe-shaped electrically conductive member; and electrically connecting each of the plurality of signal lines and each of the plurality of terminal pins by means of the pipe-shaped electrically conductive member.

Preferably, the filling the insides of both the end portions of the pipe-shaped electrically conductive member with the electrically conductive adhesive includes immersing both the end portions of the pipe-shaped electrically conductive member in a container that pools the electrically conductive adhesive.

The present invention can provide an electronic endoscope having an airtight structure that enables connection between closely packed terminal pins and signal lines.

### BRIEF DESCRIPTION OF THE DRAWINGS

Fig. 1 is a diagram for describing a configuration of an electronic endoscope;
Fig. 2 is a cross sectional view of an airtight terminal;
Fig. 3 is a plan view of the airtight terminal;
Figs. 4A to 4D are explanatory diagrams illustrating a method of connecting a terminal pin and a signal line by means of an electrically conductive member;
Figs. 5A to 5F are explanatory diagrams illustrating a method of filling a portion of the electrically conductive member on a signal line connection side with an electrically conductive adhesive and a method of connecting the electrically conductive member and the signal line;
Figs. 6A to 6D are explanatory diagrams illustrating a method of filling a portion of the electrically conductive member on a terminal pin connection side with the electrically conductive adhesive;
Fig. 7 illustrates a method of connecting the electrically conductive member and the terminal pin;
Figs. 8A to 8C are diagrams each illustrating a structure of the electrically conductive member; and
Fig. 9 is a schematic configuration diagram of an electronic endoscope.

### DETAILED DESCRIPTION OF THE PREFERRED EMBODIMENTS

Hereinafter, a preferred embodiment of the present invention is described with reference to the drawings. Although the present invention is described by way of the following preferred embodiment, the present invention can be modified according to various methods without departing from the scope of the present invention, and other embodiments than the present embodiment can be adopted. Accordingly, all modifications within the scope of the present invention are included in the scope of claims.

In the drawings, portions designated by the same reference numerals or characters are similar elements having similar functions. Herein, in the case where a numerical value range is expressed using "to", the numerical values of the upper limit and the lower limit indicated by "to" are also included in the numerical value range.

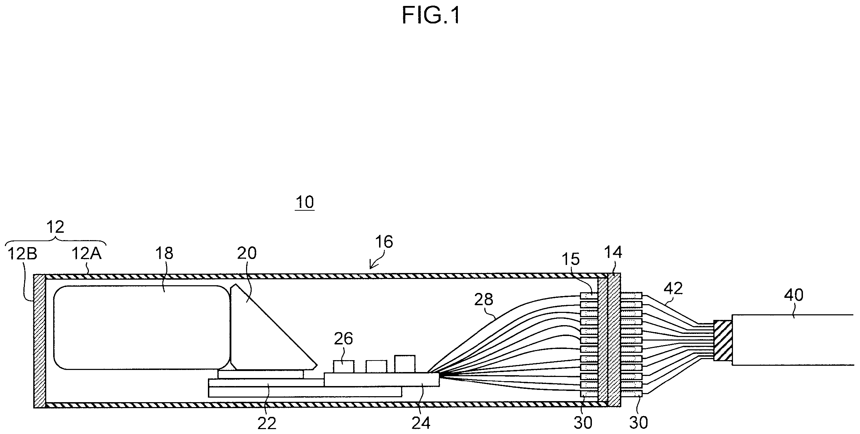

Fig. 1 is a diagram for describing a configuration of an electronic endoscope according to the present embodiment. An electronic endoscope 10 includes an airtight container 12 and an airtight terminal 14, and the airtight container 12 and the airtight terminal 14 form an airtight structure 16. The internal space of the airtight structure 16 is airtightly sealed. Here, the hermetic sealing refers to such sealing that does not allow water vapor to enter the internal space of the airtight structure 16 at the time of high-pressure high-temperature steam sterilization using an autoclave.

The airtight container 12 includes a metal tube 12A made of SUS (stainless steel) and a distal window 12B fixed to a distal end part thereof. Instead of SUS, the metal tube 12A can be made of kovar (trademark of Carpenter Technology Corporation), titanium, and the like. The distal window 12B is made of transparent sapphire, quartz, and the like. The distal end of the metal tube 12A and the distal window 12B are joined to each other by high-melting-point glass and the like. This can keep the airtight state.

The airtight terminal 14 is attached to the airtight container 12 on the side opposite to the distal window 12B. The abutting surfaces of the airtight container 12 and the airtight terminal 14 are jointed to each other by resistance welding, fusion welding, brazing, and the like. This can keep the airtight state.

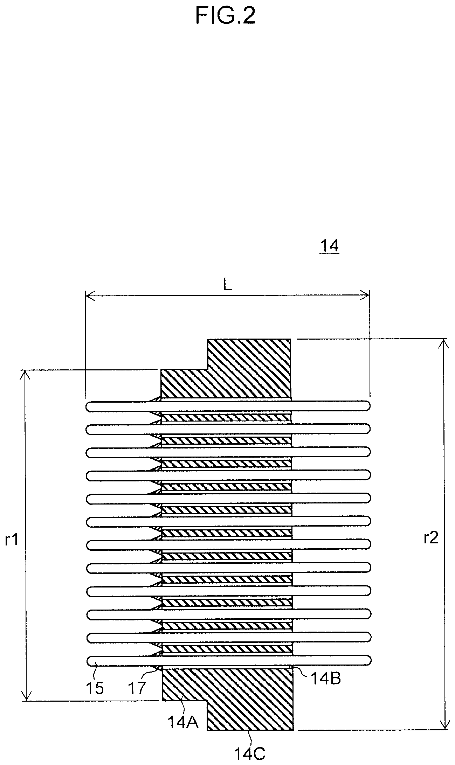

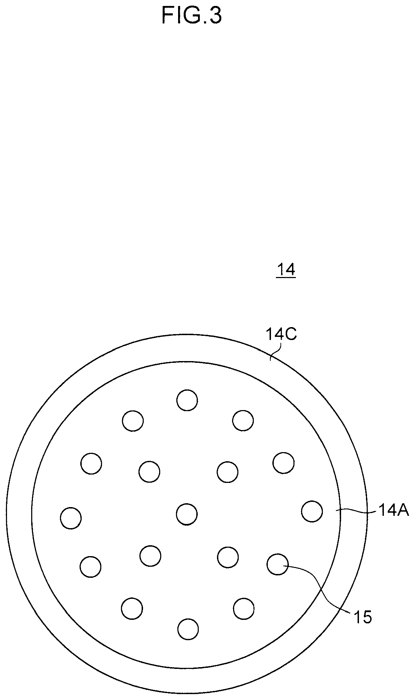

As illustrated in a cross sectional view of Fig. 2, the airtight terminal 14 includes: a base 14A that has through-holes 14B formed therein and is made of kovar; a plurality of terminal pins 15 that are respectively placed in the through-holes 14B and are made of kovar; and a sealing member 17 that fixes the terminal pins 15 to the base 14A and is made of borosilicate glass and the like. This configuration enables the airtight terminal 14 to keep the airtight state. The base 14A has a cylindrical shape, and includes a flange part 14C in an outer circumferential part thereof. The metal tube 12A abuts against the flange part 14C, and is joined thereto. The base 14A has a diameter r1 of 2.5 to 3.0 mm, and the flange part 14C has a diameter r2 of 2.8 to 3.3 mm. Each terminal pin 15 has a length L of 3 to 5 mm, and has a diameter of 0.1 to 0.3 mm. As illustrated in a plan view of Fig. 3, for example, 17 terminal pins 15 are placed in the base 14A. The interval between adjacent terminal pins 15 is 0.15 to 0.25 mm. Instead of kovar, the base 14A can be made of borosilicate glass and the like. Moreover, instead of kovar, the terminal pins 15 can be made of copper, brass, and the like. Moreover, instead of borosilicate glass, the sealing member 17 can be made of a ceramic sealing material and the like.

As illustrated in Fig. 1, an optical objective lens 18, a triangular prism 20, and an image pickup element 22 are placed in the internal space of the airtight structure 16. The triangular prism 20 orthogonally changes the optical path of incident light (observation light) that comes from an object and is taken in through the optical objective lens 18. The image pickup element 22 receives the incident light from the optical objective lens 18. The image pickup element 22 is mounted on a circuit board 24. A drive circuit component 26 for driving and controlling the image pickup element 22 is mounted on the circuit board 24. The circuit board 24 on which the drive circuit component 26 is mounted is placed in the internal space of the airtight structure 16. In order to output, to the outside, image signals based on image information of the object taken in by the image pickup element 22, a plurality of internal signal lines 28 are electrically connected to the circuit board 24.

In the present embodiment, the optical objective lens 18 is placed in the internal space of the airtight structure 16, but the present invention is not limited thereto. The optical objective lens 18 can be placed outside of the airtight structure 16.

Each of the plurality of internal signal lines 28 is electrically connected to a portion of each of the plurality of terminal pins 15 located inside of the airtight structure 16, via each of pipe-shaped electrically conductive members 30.

A signal line cable 40 is placed outside of the airtight structure 16. The signal line cable 40 includes a plurality of signal lines 42. The image information outputted from the image pickup element 22 is transmitted to a processor (not illustrated) through the signal line cable 40, and is processed into an image for display.

Each of the plurality of signal lines 42 is electrically connected to a portion of each of the plurality of terminal pins 15 located outside of the airtight structure 16, via each of the pipe-shaped electrically conductive members 30.

The electrically conductive member 30 is made of, for example, a pipe material that is made of copper and has a length of 5 to 20 mm, an inner diameter of 0.13 to 0.33 mm, and an outer diameter of 0.18 to 0.38 mm.

In the present embodiment, the terminal pins 15 and the internal signal lines 28, as well as the terminal pins 15 and the signal lines 42, are electrically connected via the pipe-shaped electrically conductive members 30.

According to the present embodiment, after the terminal pins 15 are brought into contact with the internal signal lines 28 and the signal lines 42, swaging work for fixing the two does not need to be performed. That is, it is possible to avoid difficulty in using a special tool and the like in gaps between the closely packed adjacent terminal pins 15 having extremely small diameters.

If swaging work is performed after the terminal pin 15 is inserted into the electrically conductive member 30, there is a risk that force is applied in a direction in which the terminal pin 15 may be inclined, during the swaging work. In this case, the sealing member 17 in a root portion of the terminal pin 15 cracks. If even a slight crack occurs, the airtight state cannot be secured.

Moreover, if swaging work is performed after the terminal pin 15 is inserted into the electrically conductive member 30, there is a risk that the electrically conductive member 30 may be bent during the swaging work. In this case, because the interval between the terminal pins 15 is short, if some of the electrically conductive members 30 are bent so as to approach each other, the electrically conductive members 30 thus bent come into contact with (short-circuit) each other.

According to the present embodiment, the swaging work after each terminal pin 15 is inserted into each electrically conductive member 30 is omitted, and hence a crack of the sealing members 17 and short-circuiting between the terminal pins 15 can be prevented.

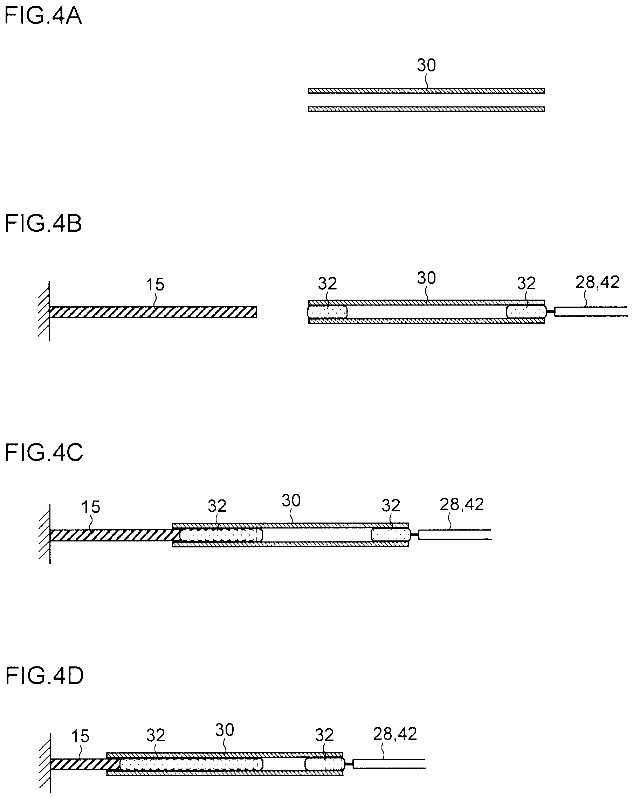

Next, a method of connecting each terminal pin 15 and each signal line 42 (internal signal line 28), which is part of a method of manufacturing the electronic endoscope, is described with reference to Figs. 4A to 4D. First, the pipe-shaped electrically conductive member 30 is prepared (Fig. 4A).

Next, the inside of one end of the electrically conductive member 30 is filled with an electrically conductive adhesive 32. The signal line 42 (internal signal line 28) is inserted into the one end of the electrically conductive member 30. The electrically conductive adhesive 32 is hardened at room temperature for about 24 hours, whereby the signal line 42 (internal signal line 28) is connected and fixed to the inside of the electrically conductive member 30. The inside of the other end of the electrically conductive member 30, with the one end to which the signal line 42 (internal signal line 28) is connected and fixed, is filled with the electrically conductive adhesive 32. The electrically conductive member 30 is moved closer to the terminal pin 15 (Fig. 4B).

The terminal pin 15 is inserted into the other end of the electrically conductive member 30 by moving the electrically conductive member 30 and/or the terminal pin 15 (Fig. 4C).

Lastly, the electrically conductive adhesive 32 is hardened at room temperature for about 24 hours, whereby the terminal pin 15 is connected and fixed to the inside of the electrically conductive member 30 (Fig. 4D). Examples of the electrically conductive adhesive 32 include: Aremco-Bond 525 (trademark) (upper temperature limit: 170°C) and Aremco-Bond 556 (trademark) (upper temperature limit: 170°C), which are produced by Aremco Products, Inc.; and Duralco 120 (trademark) (upper temperature limit: 260°C), which is produced by Cotronics Corp.

The use of the electrically conductive adhesive 32 having a high heat resistance (upper temperature limit: 130°C or higher) enables adhesivity to be maintained even under high-temperature environments. Thus, the electronic endoscope 10 can be applied to high-pressure high-temperature steam sterilization (autoclave).

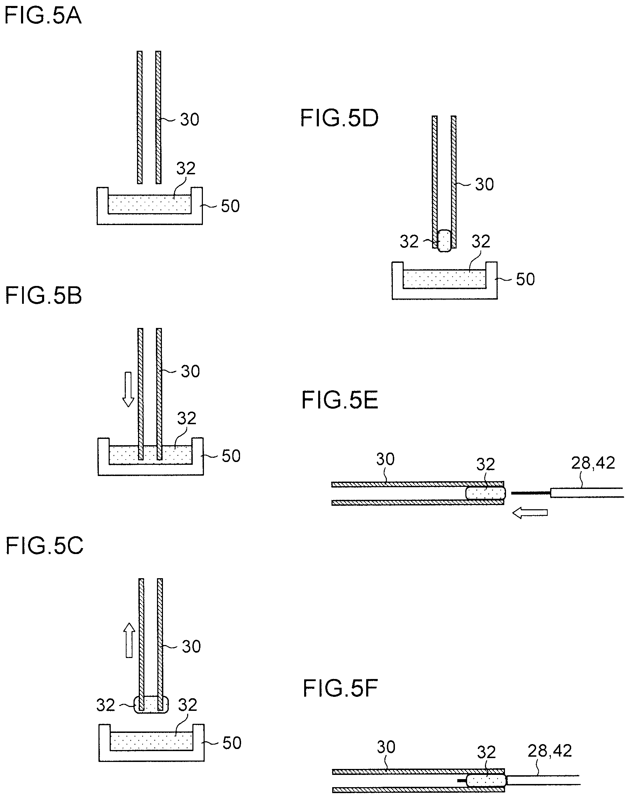

Next, a method of filling a portion of the electrically conductive member 30 on the signal line connection side with the electrically conductive adhesive 32 and a method of connecting each electrically conductive member 30 and each signal line 42 (internal signal line 28) are described with reference to Figs. 5A to 5F. The electrically conductive member 30 and a container 50 that pools (accumulates) the electrically conductive adhesive 32 are prepared (Fig. 5A). The distal end part of the electrically conductive member 30 is immersed in the electrically conductive adhesive 32 (Fig. 5B). The electrically conductive member 30 is taken out of the container 50. The electrically conductive adhesive 32 adheres to the inside and the outside of the electrically conductive member 30 (Fig. 5C). The electrically conductive adhesive 32 that adheres to the outside of the electrically conductive member 30 is wiped out. Consequently, the inside of the distal end part of the electrically conductive member 30 can be filled with the electrically conductive adhesive 32 (Fig. 5D). The wire of the signal line 42 (internal signal line 28) is inserted into the portion of the electrically conductive member 30 filled with the electrically conductive adhesive 32 (Fig. 5E). Lastly, the electrically conductive adhesive 32 is hardened at room temperature for about 24 hours, whereby the signal line 42 (internal signal line 28) is connected and fixed to the inside of the electrically conductive member 30 (Fig. 5F). In the embodiment of Figs. 5A to 5F, the case where the electrically conductive adhesive 32 is used is described, but the present invention is not limited thereto. The signal line 42 (internal signal line 28) can also be connected and fixed by soldering to the inside of the electrically conductive member 30.

The electrically conductive member 30 is immersed into the electrically conductive adhesive 32 at a fixed depth, whereby the amount of filling the electrically conductive member 30 with the electrically conductive adhesive 32 can be fixed.

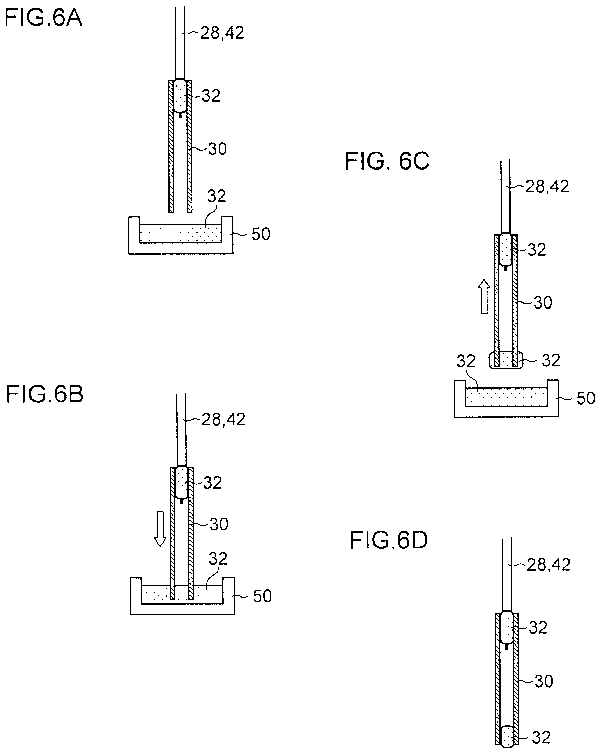

Next, a method of filling a portion of the electrically conductive member 30 on the terminal pin connection side with the electrically conductive adhesive 32 is described with reference to Figs. 6A to 6D. The electrically conductive member 30 to which the signal line 42 (internal signal line 28) is connected and fixed and the container 50 that pools the electrically conductive adhesive 32 are prepared (Fig. 6A). The distal end part of the electrically conductive member 30 is immersed in the electrically conductive adhesive 32 (Fig. 6B). The electrically conductive member 30 is taken out of the container 50. The electrically conductive adhesive 32 adheres to the inside and the outside of the electrically conductive member 30 (Fig. 6C). The electrically conductive adhesive 32 that adheres to the outside of the electrically conductive member 30 is wiped out. Consequently, the inside of the distal end part of the electrically conductive member 30 can be filled with the electrically conductive adhesive 32 (Fig. 6D).

In this way, the electrically conductive member 30, with the one end to which the signal line 42 (internal signal line 28) is connected and fixed and the other end filled with the electrically conductive adhesive 32, can be prepared. After that, the electrically conductive member 30 and the terminal pin 15 can be connected and fixed to each other according to the method illustrated in Figs. 4A to 4D.

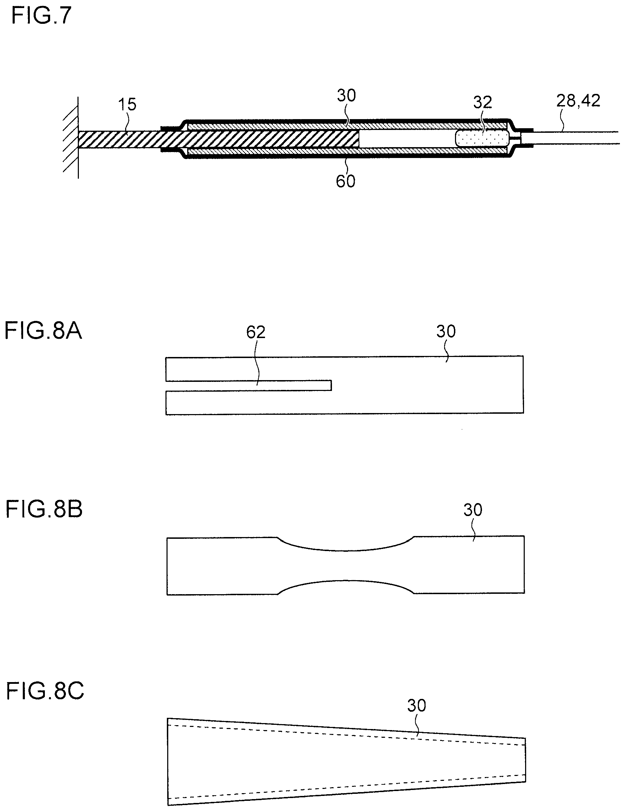

Another method of connecting each terminal pin 15 and each electrically conductive member 30 is described with reference to Fig. 7. The signal line 42 (internal signal line 28) is connected and fixed to the inside of the electrically conductive member 30 by the electrically conductive adhesive 32. The outer circumference of the electrically conductive member 30 is coated with a heat-shrinkable tube 60. In this state, the terminal pin 15 is inserted into the electrically conductive member 30. The heat-shrinkable tube 60 is heated. The heat-shrinkable tube 60 is shrunk by the heating, so that the electrically conductive member 30 and the terminal pin 15 are connected and fixed to each other. Examples of the material usable for the heat-shrinkable tube 60 include PTFE (polytetrafluoroethylene) and FEP (copolymers of tetrafluoroethylene and hexafluoropropylene). The heat-shrinkable tube 60 has insulating properties, and hence the use of the heat-shrinkable tube 60 can prevent short-circuiting between the adjacent terminal pins 15.

Another method of connecting each terminal pin 15 and each electrically conductive member 30 is described with reference to Figs. 8A to 8C. In the present embodiment, the pipe-shaped electrically conductive member 30 is subjected to processing. The electrically conductive member 30 illustrated in Fig. 8A includes a splitting slit 64 on the terminal pin 15 insertion side. The splitting slit 64 sandwiches the terminal pin 15, whereby the terminal pin 15 and the electrically conductive member 30 can be connected and fixed to each other.

The electrically conductive member 30 illustrated in Fig. 8B has an inner diameter that is smaller in the center than at both the ends. The terminal pin 15 can be sandwiched by the portion having the smaller inner diameter in the center of the electrically conductive member 30. In this way, the terminal pin 15 and the electrically conductive member 30 can be connected and fixed to each other.

The electrically conductive member 30 illustrated in Fig. 8C has a tapered shape having an inner diameter that gradually becomes smaller from the terminal pin 15 connection side toward the opposite side. Because the electrically conductive member 30 has the tapered shape, when the terminal pin 15 is inserted into the electrically conductive member 30, the terminal pin 15 can be sandwiched by the tapered part of the electrically conductive member 30. In this way, the electrically conductive member 30 and the terminal pin 15 can be connected and fixed to each other.

The shape of the electrically conductive member 30 is not limited to the above-mentioned shapes. For example, the electrically conductive member 30 may have a C shape in cross section with a slit extending from one end to the other end in the length direction of the electrically conductive member 30, and may have a spiral tube shape formed by winding a metal band in a spiral manner.

It is preferable that the electrically conductive member 30 have a surface that has been subjected to an insulation process. This can prevent short-circuiting between the adjacent terminal pins 15. Here, the insulation process refers to insulating varnish coating, coating with an insulating tube, and the like.

Description is given above of the case where the electrically conductive members 30 and the terminal pins 15 are individually connected and fixed to each other one by one, but the present invention is not limited thereto. The plurality of electrically conductive members 30 aligned using a jig or the like may be respectively inserted into the plurality of terminal pins 15 at the same time.

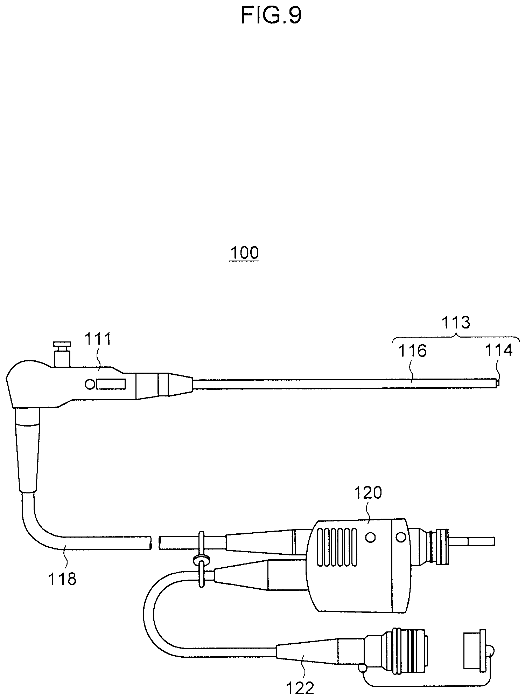

Fig. 9 is an overall configuration diagram of an endoscope apparatus according to the embodiment of the present invention. The electronic endoscope 10 includes an operation part 111 and an insertion part 113 that is provided so as to be continuous with the operation part 111 and is to be inserted into a body cavity of a patient. The insertion part 113 includes a distal end part 114 and a rigid part 116.

A universal cable (LG flexible part) 118 is connected to the operation part 111. An LG connector (LG: Light Guide) 120 is provided at the distal end of the universal cable 118. The LG connector 120 is detachably coupled to a light source apparatus (not illustrated). With this configuration, illumination light is sent from the light source apparatus to an illumination optical system built in the distal end part 114 of the insertion part 113. Moreover, a video connector 122 is also connected to the LG connector 120. The video connector 122 is detachably coupled to the processor (not illustrated) that performs image signal processing and the like.

## Claims

1. An electronic endoscope (10) comprising:
a plurality of internal signal lines (28) which are electrically connected to an image pickup element (22) that receives incident light, a circuit board (24) on which the image pickup element (22) and a drive circuit component (26) for the image pickup element (22) are mounted;
an airtight structure (16) including an airtight container (12) and an airtight terminal (14) through which a plurality of terminal pins (15) penetrate, the airtight structure (16) in which the image pickup element (22), the circuit board (24) and the plurality of the internal signal lines (28) are sealed; and
a signal line cable (40) including a plurality of signal lines (42), the signal line cable (40) being placed outside of the airtight structure (16),
the electronic endoscope (10) further comprising
a plurality of electrically conductive members (30) which electrically connect the plurality of signal lines (42) of the signal line cable (40) and the plurality of terminal pins (15) placed outside of the airtight structure (16), repectively,
**characterized in that** the electrically conductive members (30) are pipe shaped.

2. The electronic endoscope (10) according to claim 1, further comprising
an electrically conductive adhesive (32) which is used for at least one of
a connection between the signal line (42) and the electrically conductive member (30), and
a connection between the terminal pin (15) located outside of the airtight structure (16) and the electrically conductive member (30).

3. The electronic endoscope (10) according to claim 1 or 2, further comprising
an insulating tube that coats the signal line (42), the terminal pin (15) located outside of the airtight structure (16) and the electrically conductive member (30).

4. The electronic endoscope (10) according to any one of claims 1 to 3, further comprising
a plurality of pipe-shaped electrically conductive members (30) which electrically connect the plurality of internal signal lines (28) and the plurality of terminal pins (15) located inside of the airtight structure (16), respectively.

5. The electronic endoscope (10) according to claim 4, further comprising
an electrically conductive adhesive (32) which is used for at least one of
a connection between the internal signal line (42) and the electrically conductive member (30), and
a connection between the terminal pin (15) located inside of the airtight structure (16) and the electrically conductive member (30).

6. The electronic endoscope (10) according to claim 4 or 5, further comprising
a heat-shrinkable tube (60) that coats the internal signal line (42), the terminal pin (15)located inside of the airtight structure (16), and the electrically conductive member (30).

7. The electronic endoscope (10) according to claim 2 or 5, wherein the electrically conductive adhesive (32) has an upper temperature limit of 130°C or higher.

8. The electronic endoscope (10) according to any one of claims 1 to 7, wherein the electrically conductive member (30) includes a splitting slit (64) on a side to which the terminal pin (15) is connected.

9. The electronic endoscope (10) according to any one of claims 1 to 7, wherein the electrically conductive member (30) has an inner diameter that is smaller in a center than at both ends.

10. The electronic endoscope (10) according to any one of claims 1 to 7, wherein the electrically conductive member (30) has a tapered shape having an inner diameter that gradually becomes smaller from a side to which the terminal pin (15) is connected toward a side opposite thereto.

11. The electronic endoscope (10) according to any one of claims 1 to 10, wherein the electrically conductive member (30) has a surface that has been subjected to an insulation process.

12. A method of manufacturing an electronic endoscope (10), comprising:
preparing an image pickup element (22) that receives incident light, a circuit board (24) on which the image pickup element (22) and a drive circuit component (26) for the image pickup element (22) are mounted, a plurality of internal signal lines (28), an airtight terminal (14) through which a plurality of terminal pins (15) penetrate, an airtight container (12), and a signal line cable (40) including a plurality of signal lines (42);
airtightly sealing the image pickup element (22), the circuit board (24), and the plurality of internal signal lines (28) by means of the airtight terminal (14) and the airtight container (12); and
electrically connecting each of the plurality of signal lines (42) and each of the plurality of terminal pins (15) by means of a electrically conductive member (30),
**characterized in that** the electrically conductive member (30) is pipe-shaped.

13. The method of manufacturing an electronic endoscope (10) according to claim 12, further comprising:
electrically connecting the plurality of internal signal lines (28) and the circuit board (24); and
electrically connecting each of the plurality of internal signal lines (28) and each of the plurality of terminal pins (15) by means of a pipe-shaped electrically conductive member (30).

14. The method of manufacturing an electronic endoscope (10) according to claim 12 or 13, further comprising
filling insides of both end portions of the pipe-shaped electrically conductive member (30) with an electrically conductive adhesive (32), and
after the filling with the electrically conductive adhesive (32), performing at least one of:
electrically connecting each of the plurality of internal signal lines (28) and each of the plurality of terminal pins (15) by means of the pipe-shaped electrically conductive member (30); and
electrically connecting each of the plurality of signal lines (42) and each of the plurality of terminal pins (15) by means of the pipe-shaped electrically conductive member (30).

15. The method of manufacturing an electronic endoscope (10) according to claim 14, wherein the filling the insides of both the end portions of the pipe-shaped electrically conductive member (30) with the electrically conductive adhesive (32) includes immersing both the end portions of the pipe-shaped electrically conductive member (30) in a container (50) that pools the electrically conductive adhesive (32).

## Patentansprüche

1. Elektronisches Endoskop (10), umfassend:
mehrere interne Signalleitungen (28), die elektrisch an ein Bildaufnahmeelement (22) angeschlossen sind, das einfallendes Licht aufnimmt, ferner an eine elektrische Schaltungsplatine (24) angeschlossen sind, auf der das Bildaufnahmeelement (22) sowie eine Treiberschaltungskomponente (26) für das Bildaufnahmeelement (22) angebracht sind;
eine luftdichte Struktur (16), enthaltend einen luftdichten Behälter (12) und einen luftdichten Anschluss (14), durch den mehrere Anschlussstifte (15) hindurchgehen, wobei in der luftdichten Struktur (16) das Bildaufnahmeelement (22), die Schaltungsplatine (24) und die mehreren inneren Signalleitungen (28) abgedichtet sind; und
ein Signalleitungskabel (40), enthaltend mehrere Signalleitungen (42), wobei das Signalleitungskabel (40) sich außerhalb der luftdichten Struktur (16) befindet,
weiterhin umfassend:
mehrere elektrisch leitende Elemente (30), die elektrisch die mehreren Signalleitungen (42) des Signalleitungskabels (40) und die mehreren Anschlussstifte (15) außerhalb der luftdichten Struktur (16) verbinden,
**dadurch gekennzeichnet, dass** die elektrisch leitenden Elemente (30) röhrchenförmig ausgebildet sind.

2. Elektronisches Endoskop (10) nach Anspruch 1, weiterhin umfassend:
einen elektrisch leitenden Klebstoff (32), der für mindestens eine der folgenden Verbindungen verwendet wird:
eine Verbindung zwischen der Signalleitung (42) und dem elektrisch leitenden Element (30), und
eine Verbindung zwischen dem Anschlussstift (15) außerhalb der luftdichten Struktur (16) und dem elektrisch leitenden Element (30).

3. Elektronisches Endoskop (10) nach Anspruch 1 oder 2, weiterhin umfassend:
einen Isolierschlauch, der die Signalleitung (42), den außerhalb der luftdichten Struktur (16) gelegenen Anschlussstift (15) und das elektrisch leitende Element (30) bedeckt.

4. Elektronisches Endoskop (10) nach einem der Ansprüche 1 bis 3, weiterhin umfassend:
mehrere röhrchenförmige, elektrisch leitende Elemente (30), die die mehreren internen Signalleitungen (28) und die mehreren, innerhalb der luftdichten Struktur (16) befindlichen Anschlussstifte (15) elektrisch verbinden.

5. Elektronisches Endoskop (10) nach Anspruch 4, weiterhin umfassend:
einen elektrisch leitenden Klebstoff, der für mindestens eine der folgenden Verbindungen verwendet wird:
eine Verbindung zwischen der internen Signalleitung (42) und dem elektrisch leitenden Element (30), und
eine Verbindung zwischen den Anschlussstiften (15), die sich im Inneren der luftdichten Struktur (16) befinden, und dem elektrisch leitenden Element (30).

6. Elektronisches Endoskop (10) nach Anspruch 4 oder 5, weiterhin umfassend:
einen wärme-schrumpfbaren Schlauch (60), der die interne Signalleitung (42), den im Inneren der luftdichten Struktur (16) befindlichen Anschlussstift (15) und das elektrisch leitende Element (30) abdeckt.

7. Elektronisches Endoskop (10) nach Anspruch 2 oder 5, bei dem der elektrisch leitende Klebstoff (32) eine obere Temperaturgrenze von 130°C oder höher aufweist.

8. Elektronisches Endoskop (10) nach einem der Ansprüche 1 bis 7, bei dem das elektrisch leitende Element (30) einen Spaltschlitz (64) auf einer Seite, an der der Anschlussstift (15) angeschlossen ist, enthält.

9. Elektronisches Endoskop (10) nach einem der Ansprüche 1 bis 7, bei dem das elektrisch leitende Element (30) einen Innendurchmesser aufweist, der in der Mitte kleiner ist als an den beiden Enden.

10. Elektronisches Endoskop (10) nach einem der Ansprüche 1 bis 7, bei dem das elektrisch leitende Element (30) eine sich verjüngende Form aufweist, die einen Innendurchmesser besitzt, der von einer Seite, an der der Anschlussstift (15) angeschlossen ist, in Richtung der dazu entgegengesetzten Seite allmählich kleiner wird.

11. Elektronisches Endoskop (10) nach einem der Ansprüche 1 bis 10, bei dem das elektrisch leitende Element (30) eine Oberfläche besitzt, die einem Isolierprozess unterzogen wurde.

12. Verfahren zum Fertigen eines elektronischen Endoskops (10), umfassend:
Vorbereiten eines Bildaufnahmeelements (22), welches einfallendes Licht aufnimmt, einer Schaltungsplatine (24), auf der das Bildaufnahmeelement (22) und eine Treiberschaltungskomponente (26) für das Bildaufnahmeelement (22) angebracht sind, mehrere interne Signalleitungen (28), einen luftdichten Anschluss (14), durch den hindurch mehrere Anschlussstifte (15) verlaufen, eines luftdichten Behälters (12) und eines Signalkabels (40), welches mehrere Signalleitungen (42) enthält;
luftdichtes Einschließen des Bildaufnahmeelements (22), der Schaltungsplatine (24) und der mehreren internen Signalleitungen (28) mit Hilfe des luftdichten Anschlusses (14) und des luftdichten Behälters (12); und
elektrisches Verbinden jeder der mehreren Signalleitungen (42) und jeder der mehreren Anschlussstifte (15) mit Hilfe eines elektrisch leitenden Elements (30),
**dadurch gekennzeichnet, dass** das elektrisch leitende Element (30) röhrchenförmig ausgebildet ist.

13. Verfahren zum Fertigen eines elektronischen Endoskops (10) nach Anspruch 12, weiterhin umfassend:
elektrisches Verbinden der mehreren internen Signalleitungen (28) und der Schaltungsplatine (24); und
elektrisches Verbinden jeder der mehreren internen Signalleitungen (28) und jedes der mehreren Anschlussstifte (15) mit Hilfe eines röhrchenförmigen, elektrisch leitenden Elements (30).

14. Verfahren zum Fertigen eines elektronischen Endoskops (10) nach Anspruch 12 oder 13, weiterhin umfassend:
Befüllen des Inneren beider Endbereiche des röhrchenförmigen, elektrisch leitenden Elements (30) mit einem elektrisch leitenden Klebstoff (32), und
nach dem Befüllen mit dem elektrisch leitenden Klebstoff (32), Ausführen mindestens eines der folgenden Schritte:
elektrisches Verbinden jeder der mehreren internen Signalleitungen (28) und jedes der mehreren Anschlussstifte (15) mit Hilfe des röhrchenförmigen, elektrisch leitenden Elements (30); und
elektrisches Verbinden jeder der mehreren Signalleitungen (42) und jedes der merheren Anschlussstifte (15) mit Hilfe des röhrchenförmigen, elektrisch leitenden Elements (30).

15. Verfahren zum Fertigen eines elektronischen Endoskops (10) nach Anspruch 14, bei dem das Befüllen des Inneren beider Endabschnitte des röhrchenförmigen, elektrisch leitenden Elements (30) mit dem elektrisch leitenden Klebstoff das Eintauchen beider Endabschnitte des röhrchenförmigen, elektrisch leitenden Elements (30) in einen Behälter (50) umfasst, der den elektrisch leitenden Klebstoff (32) enthält.

## Revendications

1. Endoscope électronique (10), comprenant :
une pluralité de lignes de signal internes (28), lesquelles sont connectées par voie électrique à un élément de prise de vues (22), lequel reçoit une lumière incidente, une carte de circuit imprimé (24) sur laquelle sont montés l'élément de prise de vues (22) et un composant de circuit de commande (26) pour l'élément de prise de vues (22) ;
une structure étanche à l'air (16) incluant un conteneur étanche à l'air (12) et une borne étanche à l'air (14) à travers laquelle pénètrent une pluralité de broches-bornes (15), la structure étanche à l'air (16), où l'élément de prise de vues (22), la carte de circuit imprimé (24) et la pluralité de lignes de signal internes (28) sont scellées, et
un câble de ligne de signal (40), incluant une pluralité de lignes de signal (42), le câble de ligne de signal (40) étant placé hors de la structure étanche à l'air (16),
l'endoscope électronique (10) comprenant en outre :
une pluralité d'éléments conducteurs de l'électricité (30), lesquels connectent par voie électrique respectivement la pluralité de lignes de signal (42) du câble de ligne de signal (40) et la pluralité de broches-bornes (15) placées hors de la structure étanche à l'air (16),
**caractérisé en ce que** les éléments conducteurs de l'électricité (30) sont en forme de conduite.

2. Endoscope électronique (10) selon la revendication 1, comprenant en outre :
un adhésif conducteur de l'électricité (32), lequel est utilisé pour au moins une connexion parmi :
une connexion entre la ligne de signal (42) et l'élément conducteur de l'électricité (30), et
une connexion entre la broche-borne (15) située hors de la structure étanche à l'air (16) et l'élément conducteur de l'électricité (30).

3. Endoscope électronique (10) selon la revendication 1 ou 2, comprenant en outre :
un tube isolant, lequel revêt la ligne de signal (42), la broche-borne (15) située hors de la structure étanche à l'air (16), et l'élément conducteur de l'électricité (30).

4. Endoscope électronique (10) selon l'une quelconque des revendications 1 à 3, comprenant en outre :
une pluralité d'éléments conducteurs de l'électricité (30) en forme de conduite, lesquels connectent respectivement par voie électrique la pluralité de lignes de signal internes (28) et la pluralité de broches-bornes (15) situées dans la structure étanche à l'air (16).

5. Endoscope électronique (10) selon la revendication 4, comprenant en outre :
un adhésif conducteur de l'électricité (32), lequel est utilisé pour au moins une connexion parmi :
une connexion entre la ligne de signal interne (42) et l'élément conducteur de l'électricité (30), et
une connexion entre la broche-borne (15) située dans la structure étanche à l'air (16) et l'élément conducteur de l'électricité (30).

6. Endoscope électronique (10) selon la revendication 4 ou 5, comprenant en outre :
un tube thermorétractable (60), lequel revêt la ligne de signal interne (42), la broche-borne (15) située dans la structure étanche à l'air (16), et l'élément conducteur de l'électricité (30).

7. Endoscope électronique (10) selon la revendication 2 ou 5, dans lequel l'adhésif conducteur de l'électricité (32) présente une limite de température supérieure égale ou supérieure à 130 °C.

8. Endoscope électronique (10) selon l'une quelconque des revendications 1 à 7, dans lequel l'élément conducteur de l'électricité (30) inclut une fente de division (64) sur un côté sur lequel est connecté la broche-borne (15).

9. Endoscope électronique (10) selon l'une quelconque des revendications 1 à 7, dans lequel l'élément conducteur de l'électricité (30) présente un diamètre interne plus petit en un centre qu'au niveau des deux extrémités.

10. Endoscope électronique (10) selon l'une quelconque des revendications 1 à 7, dans lequel l'élément conducteur de l'électricité (30) présente une forme diminuant progressivement avec un diamètre interne qui devient progressivement plus petit d'un côté, sur lequel est connectée la broche-borne (15), à un côté opposé à celui-ci.

11. Endoscope électronique (10) selon l'une quelconque des revendications 1 à 10, dans lequel l'élément conducteur de l'électricité (30) présente une surface qui a été soumise à un processus d'isolation.

12. Procédé de fabrication d'un endoscope électronique (10) comprenant les étapes consistant à :
préparer un élément de prise de vues (22), lequel reçoit une lumière incidente, une carte de circuit imprimé (24) sur laquelle sont montés l'élément de prise de vues (22) et un composant de circuit de commande (26) pour l'élément de prise de vues (22), une pluralité de lignes de signal internes (28), une borne étanche à l'air (14) à travers laquelle pénètrent une pluralité de broches-bornes (15), un conteneur étanche à l'air (12), et un câble de ligne de signal (40) incluant une pluralité de lignes de signal (42) ;
sceller de manière étanche l'élément de prise de vues (22), la carte de circuit imprimé (24), et la pluralité de lignes de signal internes (28) au moyen de la borne étanche à l'air (14) et du conteneur étanche à l'air (12), et
connecter par voie électrique chacune de la pluralité de lignes de signal internes (42), et chacune de la pluralité des broches-bornes (15) à l'aide d'un élément conducteur de l'électricité (30),
**caractérisé en ce que** l'élément conducteur de l'électricité (30) est en forme de conduite.

13. Procédé de fabrication d'un endoscope électronique (10) selon la revendication 12, comprenant en outre les étapes consistant à :
connecter par voie électrique chacune de la pluralité de lignes de signal internes (28), et la carte de circuit imprimé (24), et
connecter par voie électrique chacune de la pluralité de lignes de signal internes (28) et chacune de la pluralité des broches-bornes (15) à l'aide d'un élément conducteur de l'électricité en forme de conduite (30).

14. Procédé de fabrication d'un endoscope électronique (10) selon la revendication 12 ou 13, comprenant en outre les étapes consistant à :
remplir les intérieurs des deux portions d'extrémité de l'élément conducteur de l'électricité (30) en forme de conduite avec un adhésif conducteur de l'électricité (32), et
après remplissage avec l'adhésif conducteur de l'électricité (32), réaliser au moins une des étapes suivantes :
connecter par voie électrique chacune de la pluralité de lignes de signal internes (28) et chacune de la pluralité des broches-bornes (15) à l'aide d'un élément conducteur de l'électricité en forme de conduite (30), et
connecter par voie électrique chacune de la pluralité de lignes de signal (42) et chacune des broches-bornes (15) à l'aide d'un élément conducteur de l'électricité en forme de conduite (30).

15. Procédé de fabrication d'un endoscope électronique (10) selon la revendication 14, dans lequel l'étape de remplissage des intérieurs des deux portions d'extrémité de l'élément conducteur de l'électricité en forme de conduite (30) avec l'adhésif conducteur de l'électricité (32) inclut l'immersion des deux portions d'extrémité de l'élément conducteur de l'électricité en forme de conduite (30) dans un conteneur (50) qui contient l'adhésif conducteur de l'électricité (32).
